# EUROPÄISCHE PATENTSCHRIFT

(11) **EP 1 299 420 B1**
(45) Veröffentlichungstag und Bekanntmachung des Hinweises auf die Patenterteilung: **13.02.2008**
(21) Anmeldenummer: 01956306.3
(22) Anmeldetag: 05.07.2001
(51) Int. Cl.: C07K 16/02, A61K 39/395, A61K 31/65, A61K 31/355, A61K 31/375, A61K 8/00

(54) **ZUSAMMENSETZUNG FÜR PHARMAKA UND KOSMETIKA**
COMPOSITION FOR PHARMACEUTICAL AND COSMETIC PRODUCTS
COMPOSITION POUR DES PRODUITS PHARMACEUTIQUES ET COSMETIQUES

(30) Priorität: 05.07.2000 DE 10033939; 02.09.2000 DE 20015177 U
(43) Veröffentlichungstag der Anmeldung: 09.04.2003
(73) Patentinhaber: Bergter, Wolfgang, Dr., 30625 Hannover (DE); Kobilke, Hartmut, Dr., 14797 Damsdorf (DE); Sroka, Joachim, 14797 Damsdorf (DE)
(72) Erfinder: KOBILKE, Hartmut, 14797 Damsdorf (DE)
(74) Vertreter: Schneider, Henry
(86) Internationale Anmeldenummer: PCT/DE2001/002589
(87) Internationale Veröffentlichungsnummer: WO 2002/002642

(56) Entgegenhaltungen:
- WO-A-88/07364
- WO-A-97/37636
- WO-A-99/04804
- DE-U- 20 012 123
- DE-U- 29 904 221
- FR-A- 2 747 922
- FR-M- 5 279
- CARLANDER DAVID ET AL: "Peroral immunotheraphy with yolk antibodies for the prevention and treatment of enteric infections." IMMUNOLOGIC RESEARCH, Bd. 21, Nr. 1, Februar 2000 (2000-02), Seiten 1-6, XP001038144 ISSN: 0257-277X
- HATTA H ET AL: "PASSIVE IMMUNIZATION AGAINST DENTAL PLAQUE FORMATION IN HUMANS: EFFECT OF A MOUTH RINSE CONTINGIN EGG YOLK ANTIBODIES (IgY) SPECIFIC TO STREPTOCOCCUS MUTANS" CARIES RESEARCH, S. KARGER AG, BASEL, CH, Bd. 31, Nr. 4, 1. Juli 1997 (1997-07-01), Seiten 268-274, XP002084520 ISSN: 0008-6568
- SUGITA-KONISHI YOSHIKO ET AL: "Immune functions of immunoglobulin Y isolated from egg yolk of hens immunized with various infectious bacteria." BIOSCIENCE BIOTECHNOLOGY AND BIOCHEMISTRY, Bd. 60, Nr. 5, 1996, Seiten 886-888, XP001038136 ISSN: 0916-8451
- DATABASE WPI Derwent Publications Ltd., London, GB; AN 92-085848 XP002188430 "Skin cosmetic with prolonged effect - contains (modified) antibody and unusual carrier " (TAKARA SHUZO CO LTD), 31. Januar 1992 (1992-01-31) & JP 04 029912 A (TAKARA SHUZO CO LTD) 31. Januar 1992 (1992-01-31)
- DATABASE WPI Derwent Publications Ltd., London, GB; AN 96-157000 XP002188432 "SKIN ADHESIVE SHEET SKIN INJURY TREAT PREPARATION DRY GEL CONTAIN POLYSACCHARIDE EXTRACT SEA ALGAE" (ICHIMARU PHARCOS INC), 13. Februar 1996 (1996-02-13) & JP 08 040882 A 13. Februar 1996 (1996-02-13)
- DATABASE WPI Derwent Publications Ltd., London, GB; AN 81-94328D XP002188431 "LIPID PRODN. FROM GREEN ALGAE - INCLUDES EXTN. WITH ALCOHOL-CHLOROFORM MIXT. AT LOW TEMP., FOR USE IN COSMETICS AND HYGIENE PRODUCTS" ((AUZM-R) AS UZB MICROBIOL), 15. März 1981 (1981-03-15) & SU 812 291 B 15. März 1981 (1981-03-15)

## Beschreibung

Die Erfindung betrifft eine Zusammensetzung für Pharmaka und Kosmetika zur Behandlung von sowohl akuten als auch antibiotikaresistenten chronischen Entzündungen.

Im folgenden wird unter Pharmaka der Oberbegriff verstanden für rezeptpflichtige und nicht rezeptpflichtige Arzneimittel, für homöopathische Arzneimittel sowie Nahrungsergänzungsmittel.

Bekannt ist eine große Anzahl verschiedener Zusammensetzungen mit unterschiedlichen spezifischen Wirkungen. Es befinden sich darunter Zusammensetzungen mit hautschützenden, hautaufbauenden und regenerierenden, entzündungshemmenden wie auch antimikrobiellen Wirkungen, die entweder antibiotisch oder im allgemein-immunologischen Sinne auf Wundkeime neutralisierend wirken und die als Wirkstoffkomponenten sowohl Naturpflanzenextrakte und/oder Immunglobuline oder Chemotherapeutika, Vitamine und/oder Antibiotika enthalten.

Eine Verwendung von IgY Antikörpern zur Behandlung von Hautinfektionen ist aus WO-A 97/37636 bekannt. Der Einsatz von Mikroalgenextrakten zur Behandlung von Wundinfektionen ist in FR-M 5279M und FR-A 2747922 beschrieben. Dabei wird in der Literatur auch die Kombination von Mikroalgenextrakten mit Antibiotika zur Erzielung synergistischer Wirkungen dargestellt (SU-B 812291 und FR-M 5279M). Auch WO-A 88/07364 beschreibt Oxytetracyclin als Bestandteil einer Wundsalbe.

Bekannt sind weiterhin im wesentlichen vier Gruppen von Zusammensetzungen und ihre Kombinationen untereinander:
auf rein pflanzlichen Extrakten basierende Zusammensetzungen, die insbesondere gegen Bakterien und zur Unterstützung der Wundheilung, zur Zellernährung im Heilungsprozeß sowie als entzündungshemmende Komponenten zum Einsatz kommen. So ist beispielsweise aus der DE 196 34 959 A1 eine Wund- und Heilsalbe bekannt, die Ringelblumenöl und weitere pflanzliche Inhaltsstoffe aufweist. In der WO 97/42963 A2 wird eine sogenannte "grüne Salbe" zur Behandlung von Verbrennungen und Wunden beschrieben, die im wesentlichen aus pflanzlichen Bestandteilen, unter anderem Calendula, besteht und einen Salbenträger aus Olivenöl mit Honigwachs versetzt nennt. Die Wirkungen einzelner Pflanzenbestandteile werden beschrieben und die von der Wirkung betroffenen Bakterienstämme identifiziert.

- Zusammensetzungen auf chemotherapeutischer (antibiotischer) Basis, insbesondere gegen bestimmte Wundinfektionskeime polykausaler Natur, werden unter anderem in der JP 080 533 68 A mit dem Bestandteil Tetracyclin sowie in der WO 88/07364 A1 mit dem Bestandteil Oxytetracyclin beschrieben. Weitere Salben mit Bestandteilen von Linomycin und Chloramphenicol sind bekannt.
- Darüber hinaus sind diverse Salben mit einfachen Wirkungen bekannt, wie sie beispielsweise gegen Bakterien durch Zusatz von Tetracyclinen Anwendung finden. Derartige Salben sind unter anderem in den Schriften WO 8807364 A1 und JP 08053368 A beschrieben und basieren im wesentlichen auf dem Wirkstoff Ringelblumenblütenextrakt und/oder Tetracyclin.
- Auch sind Zusammensetzungen auf der Basis von allgemeinen und systemischen (inneren) Wirkungen von Serum-Immunglobulinen der Klassen IgA, IgM und IgG bekannt. So wird in der Schrift WO 94/15640 A1 die Anwendung von Immunglobulinen IgA, IgM und IgG aus Blutserum inauguriert als eine Möglichkeit zur spezifisch entzündungshemmenden Wirkung im systemischen Sinne (z.B. Sepsis), weniger im lokalen Sinne.

Die Nutzbarkeit von Eidotterantikörpern (IgY) wird in einer Reihe von Schriften wie z.B. WO 95/02612 A1; EP 0 696 596 A1; US 5 367 054 A; JP 092 392 03A; DE 196 50 991 A1 zwar beschrieben. Diese Veröffentlichungen haben aber ausschließlich Verfahren und Einsatzmöglichkeiten in der passiven Immuntherapie und Diagnostik bei Mensch und Tier zum Inhalt. Auch in der WO 98/14209 A1 wird die antünfektiöse Wirkung von IgY beschrieben, jedoch beschränkt sich diese Veröffentlichung auf die orale Verabreichung.

Eine orale Zusammensetzung, die IgA und Immunglobulin G (IgG) enthält, beschreibt das US-Patent 4 335 099 zur Behandlung von Darminfektionen. Zusätzlich sind Präparationen, die 73 % IgA und 26 % IgG enthalten, bezogen auf den Gesamtimmunglobulingehalt, in der Lage, das Auftreten nekrotisierender Enterocolitis zu vermindern, wenn sie prophylaktisch an Säuglinge mit niedrigem Geburtsgewicht verabreicht werden. Es wird angenommen, daß dieser Effekt auf der Bildung eines Antigen-Antikörperkomplexes aufgrund des hohen Antikörpertiters gegen eine Mehrzahl von potentiellen Pathogenen und Toxinen beruht. Solche Pathogene umfassen Bakterien, Pilze und auch Viren. Es wurde unter anderem in der EP 0 506 651 beschrieben, daß IgA, IgG und Transferrin synergistisch gegen Bakterienwachstum wirken. Die Anteile der Wirkstoffe liegen zwischen 0,40 und 0,80 Gew.-Teile IgG und 0,15 bis 0,45 Gew.-Teile Transferrin pro Gewichtsteil IgA.

Es wurde gezeigt, daß IgG, IgA und IgM synergistisch mit anderen pharmakologisch aktiven Verbindungen, wie Antibiotika, agieren. Diese Immunglobuline binden vermutlich an infektiöse Mikroorganismen, was zu deren Agglutination, Neutralisation oder Induktion von Phagocytose führt (EP 0 168 830).
Die Applikation der bekannten Salben ist sowohl beim Mensch als auch beim Tier bzw. Heimtier nur in Räumen mit Zimmertemperatur möglich. Es haftet also allen bekannten Salben und Emulsionen der Nachteil an, daß ihre Anwendbarkeit unter frostigen Außenbedingungen nicht möglich ist bzw. ihre Wirkung nicht oder nur unvollkommen eintritt, weil ein Eindringen der wertvollen Wirksubstanzen in die Wunden oder Entzündungsherde nicht möglich wird.
Aus dem bekannten Stand der Entwicklung geht hervor, daß im wesentlichen Zusammensetzungen auf monovalenter oder monospezifischer Wirkungsbasis (Antibiotika, entzündungshemmende pflanzliche Wirkungsbasen oder Immunglobine des Blutserums) beschrieben wurden.

Weiterhin wird von CARLANDER DAVID und anderen in "Peroral immunotherapy with yolk antibodies for the prevention and treatment of enteric infections", IMMUNOLOGIC RESEARCH, Bd 21, Nr. 1 Februar 2000, Seiten 1-6 eine perorale Immuntherapie zur Prävention oder Therapie von bakteriellen und viralen Infektionen im Mundbereich und Magen-Darm-Trakt unter Verwendung von IgY, das aus konventionellen Hennen hergestellt wird, beschrieben. Ebenso wird bei HATTA und anderen in "Passive immunization against dental plaque formation in humans: Effect of a mouth rinse containing egg yolk antibodies (IgY) specific to streptococcus mutans", CARIES RESEARCH, S. KARGER AG, BASEL, CH, Bd 31, Nr. 4, 1. Juli 1997, Seiten 268-274 eine perorale passive Immunisierung mit IgY und Malvenextrakt gegen S. mutans zur Prävention von Plaques und Karies beschrieben.
SUGITA-KONISHI YOSHIKO und andere berichten in "Immune functions of immunoglobulin Y isolaled from egg yolk of hens immunized with various infectious bacteria", BIOSCIENCE BIOTECHNOLOGY AND BIOCHEMISTRY, Bd 60, Nr. 5, 1996, Seiten 886-888 von Modellversuchen mit IgY (polyvalent), das durch Immunisierung konventioneller Hennen mit einer Mischung aus 26 Bakterienarten gewonnen wurde und beschreiben deren Wirkungen. Dabei geht es um die Prävention von Krankheiten.

Nach der DE 299 04 221 U1 wird IgY als Futterzusatz in der Tierzucht zur Prävention von Krankheiten eingesetzt. Erwähnt wird auch eine perorale passive Immunisierung mit IgY. Auch aus der WO 97/37636 A1 ist der Einsatz von IgY, das durch Immunisierung konventioneller Hennen hergestellt wurde, als Zusatz zu Lebens- bzw. Genussmitteln zur peroralen Immunisierung zur Prävention von Krankheiten bekannt.
Ein künstlicher Speichelersatz, der in erster Linie Bestandteile des Speichels des angeborenen Immunsystems enthält und alternativ auch IgY konventioneller Hennen enthält, ist aus der WO 99/ 04804 A bekannt. Erwähnt werden auch pflanzliche Extrakte in Kombination mit IgY.
In *DATABASE WPI, Derwent Publications Ltd., London, GB; AN 92-085848 XP 002188430 "Skin cosmetic with prolonged effect - contains (modified) antibody and unusual carrier"* ist ein Kosmetikum, das funktionelle Anti-Keratin-Antikörper enthält, offenbart.
Wundauflagen, die Polysaccharidextrakte aus Algen enthalten und Lipidextrakte aus Algen sind in *DATABASE WPI, Derwent Publications Ltd, London, GB; AN 96-157000 XP 002188432 "Skin adhesive sheet skin injury treat preparation dry gel contain polysaccharide extract sea algae"* bzw. *DATABASE WPI, Derwent Publications Ltd., London, GB; AN 81-94328D XP 002188431 "Lipid Prodn. from green algae - includes extn. with alcohol-chloroform mixt. at low temp., for use in cosmetics and hygiene products"* beschrieben.
Mit dem französischen Patent FR 5.279 M und der französischen Patentanmeldung FR 2 747 922 A ist eine Wundsalbe, die Extrakte von Spirulina Maxima enthält und eine kosmetische Zubereitung unter Verwendung von Naturalgenextrakten vom Typ Chlorella, bekannt geworden.

Es ist Aufgabe der Erfindung, eine pharmazeutische Zusammensetzung zur Herstellung von Pharmaka und Kosmetika in Form einer speziellen aber dennoch universal einsetzbaren Zusammensetzung zur Behandlung von akuten und hartnäckigen chronischen Entzündungsreaktionen der Haut und der kutanen Schleimhäute bei Mensch und Tier zur Verfügung zu stellen. Es ist eine weitere Aufgabe der Erfindung, Kombinationen pharmazeutischer Zusammensetzungen bereitzustellen, mit denen Mensch und Tier erfolgreicher behandelt werden können und die bei Mensch und Tier sehr schnell und in Entzündungsherde tief eindringend prophylaktisch, therapeutisch und metaphylaktisch gegenüber Wundinfektionen wirkend einsetzbar sind.
Die vorzuschlagende Zusammensetzung soll in flüssiger, pastöser oder fester Form in verschiedensten Pharmaka (beispielsweise Lotionen, Wundsprays, Salben oder Puder) sowie in Nahrungsergänzungsmitteln (beispielsweise als Saft, Tablette oder Kapsel) einsetzbar sein.

Der Erfindung liegen die Erkenntnisse über die monospezifischen Wirkungen der EinzeIkomponenten sowie ihrer kombinatorisch-synergistischen Wirkungen auf insbesondere schlecht heilende, infizierte Wunden unter allen Witterungsbedingungen zugrunde. Die polyvalente Zusammensetzung und ihre komplexe, synergistische Heilwirkung in Richtung antiphlogistisch, antimikrobiell und Haut-Gewebe regenerierend basiert auf der erfindungsgemäßen Kombination von
mono- oder polyvalenten, polyklonalen, spezifischen Eidotterantikörpern von SPF-und/oder Vorzugshennen unter Semi-SPF-Haltungsbedingungen (Status definiert), gehalten unter tiergerechten Bedingungen und mit einer definierten Zuchthennenmischung gefüttert. Die für die Antikörper wichtigen Ausgangsprodukte, die Eidotter, sind wie das gesamte Ei rückstands- und fremdkontaminationsfrei und zertifizierbar. Die gesamte Produktionsstrecke (-kette), ausgehend vom Spenderhennenbestand, Immunisierungen und Aufbereitung werden begleitet von den Zertifiziemngen "EUÖKO-Audit", EU-IS09001 und GLP-Zertifikat. Die Antikörper entfalten spezifische Wirkungen gegen die relevanten Wundinfektionskeime (Bakterien) und gegen Antibiotika- und Desinfektionsmittel-resistente Bakterien (Hospitalismusproblem),
- Ringelblütenextrakt
- Mikroalgenextrakt, vorzugsweise -öle, in den Variationen wäßriger, ethanolischer und CO₂-Extrakte von Spirulina platensis sowie
- Antibiotika, vorzugsweise Oxytetracyclin.

Die erfindungsgemäße Zusammensetzung umfasst die wesentlichen Wirkbestandteile 4,0 bis 10,0 Gewichtsteile IgY-Eidotter-(mono- der polyvalent) vom SPF-Huhn, 2,0 bis 5,0 Gewichtsteile Mikroalgenextrakte von Spirulinaarten und/oder Chlorellaarten und 3,0 bis 5,0 Gewichtsteile Ringelblumenblütenextrakt. Sie kann weiterhin Oxytetracyclinum sowie α-Tokopherol als Antioxidans und Ascorbinsäure als natürliches Konservierungsmittel enthalten. Bei Pharmaka in Form von Salben enthält sie weiterhin Paraffinum subliquidum, Lanolinum (Paraffinum subliquidum 15,0; Aqua 20,0; ceralanae ad 100,0), Ringelblumenextrakt (Extractum Calendulae fluid 1:2/35%), Oxytetracyclinum, oder alternativ ohne Oxytetracyclinum sowie α-Tokopherol als Antioxidans und Ascorbinsäure als natürliches Konservierungsmittel.

Diese Zusammensetzung ist für Mensch und Tier zur Wundbehandlung vorgesehen, auch bei hartnäckig eiternden, entzündeten abszedierenden, gangränös-nekrotisierenden Haut- und Schleimhautwunden, infizierten Gangränen bei Menschen, zur therapeutischen Behandlung bei Verbrennungen, Verätzungen, Verbrühungen, Erfrierungen der Haut und der äußerlich erreichbaren mukösen Schleimhäute von Ohr, Mundhöhle, Genital- und Analbereich, zur Behandlung von Furunkeln und nässenden Hautekzemen, bei Dekubitus und bei Wundinfektionen mit multiresistenten Bakterien (Keimen) sowie bakteriellen Wundinfektionen.

Die vorteilhaften Merkmale der Erfindung gehen außer aus den Ansprüchen auch aus den Ausführungsbeispielen hervor, wobei die einzelnen Merkmale jeweils für sich allein oder zu mehreren in Form von Unterkombinationen schutzfähige Ausführungen darstellen, für die hier Schutz beansprucht wird. Die Erfindung wird im folgenden anhand der Zusammensetzung einer Heil- und Wundsalbe näher erläutert.

Nachfolgend sind die eingesetzten Wirk- und Präparatekomponenten sowie ihre Zusammensetzung und Charakteristik erläutert:

### 1. Salbenbasis

Ein wesentlicher Bestandteil der erfindungsgemäßen pharmazeutischen Zusammensetzung in Form einer Heil- und Wundsalbe ist die gewählte Zusammensetzung der Salbenbasis Lanolinum und Paraffinum subliquidum, die bewirkt, daß sich die auf die Haut und/oder die Schleimhäute aufgebrachte Salbe sofort verflüssigt und dadurch mit ihren spezifischen Wirksfoffen bis tief in das Wund- bzw. Entzündungsgewebe penetriert.
- Lanolin ist eine neutrale, aus Wollwachs (Ceralanae) 65 v. H., Wasser 20 v. H. und Paraffinum subliquidum 15 v. H. hergestellte, homogene, pastöse, gut verträgliche Salbengrundlage.
- Paraffinum subliquidum (dickflüssiges Paraffin) wird zwischen 25 - 30 v. H. zusätzlich als klare, farb- und geruchslose ölige Flüssigkeit gesättigter und gereinigter Kohlenwasserstoffe der Salbe beigemischt mit dem Ziel, daß sich diese sofort beim Aufstreichen auf körperwarme Haut oder kutane Schleimhaut verflüssigt. Diese Eigenschaft bewirkt das tiefe Penetrieren der Wunde und damit auch die gute Tiefenwirkung der Salbeninhaltsstoffe, insbesondere der antibiotischen, antimikrobiell-immunisierenden und nutritiv-regenerativen Wirkkomponenten.

### 2. Eidotterantikörper (IgY):

Die spezifischen Eidotterantikörper mit mono- oder polyvaienter spezifischer Wirkung und Zusammensetzung wurden aus den Eidottern bzw. Dotterextrakten solcher Art immunisierter SPF- und/oder Vorzugshennen gewonnen, die gegen Staphylococcus aureus, Citrobacter, Enterobacteriacege, Mikro- und Streptokokken, Corynebakterien/coryneforme (incl. Actionomyces), coliforme (incl. Klebsiella), Neisserien, multiresistente Staphylokokken sowie weitere, aktuelle, relevante Erreger-Stämme in infizierten Wunden bzw. im Umfeld gerichtet sind.

Diese spezifischen polyklonalen Antikörper reagieren lokal in Wundgeweben und aufWundoberflächen immunologisch auf Wundantigene im Sinne einer Antigen-Antikörperreaktion; sie neutralisieren die Wundinlektionskeime spezifisch, verstärken die Makrophagenaktivität und verhindern eine weitere Keimvermehrung und deren gewebezerstörende Wirkung. Die spezifischen Eidotterantikörper wurden zu 4 bis 10 v. H. in den Salbenserien zugesetzt.

### 3. Ringelblumenblütenextrakt:

Ringelblumenblütenextrakt (Extractum Calendulae fluid 1:2, 35 v. H.) in flüssiger Zubereitung als ethanolischer oder wäßriger Extrakt dient als Antiphlogistikum, besitzt also eine gute entzündungshemmende Wirkung auf Wunden und Gewebe und wird zwischen 3,0 bis 5,0 v. H. der Salbe zugemischt. Die wesentlichen Wirkstoffe sind Triterpensaponine und -alkohole, Carotinoide, ätherische Öle und Polysaccharide. Allergieauslösende Nebenwirkungen wurden bei den Patienten bisher nicht beobachtet.

### 4. Mikroalgenextrakte:

Mikroalgenextrakte, beispielsweise von Spirulina platensis, sind jeweils zu Anteilen von 2 bis 5 v. H. in der erfindungsgemäßen Zusammensetzung in Form von wäßrigen (aqua), alkoholischen (ethanol.) oder CO₂-Extrakten enthalten. Aufgrund der Vielfalt an hochwertigen Inhaltsstoffen wie
- hohen Proteingehalten mit dem kompletten Spektrum an essentiellen und nichtessentiellen Aminosäuren
- hochmolekularen und komplex zusammengesetzten Polysacchariden
- Fetten und Fettsäuren, insbesondere Linol-, Linolen-, Omega-3- und Omega-6-Fettsäuren
- Mineralstoffen und Spurenelementen (Ca, P, FE, MG, Zn, Cr und Se)
- Pigmenten (Chlorophyll, Carotinoide) und Phytoenzymen sowie
- Vitaminen als Biokatalysatoren (E, C, B-Komplex)
besitzen diese Extrakte eine hervorragende gesundheitsfördernde und Wundgewebe regenerierende Wirkung. Die Mineralien, Spurenelemente, Enzyme, Fettsäuren sowie Vitamine fördern den Stoffwechsel und die Durchblutung und haben somit einen wesentlichen und beschleunigenden Anteil an der Wundheilung durch Erkrankungsverkürzung und geringe Wundnachsorge. Von besonderem Wert sind die ungesättigten Fettsäuren in ihrer Unterstützungsfunktion als Entzündungshemmer und Beschleuniger der Wundheilung. Die Polysaccharide aktivieren (auch lokal) die Immunglobulin- und Interleukinsynthese und -wirkung.

### 5. Oxytetracyclin (OTC):

OTC dient unter Zusatz von 2 v. H. zur Salbe als bewährtes Breitband-Antibiotikum, insbesondere gegen mikrobielle, bakterielle Wundinfektionen, vor allem gegen grampositive Bakterien sowie gegen Pilze und Hefen im Wundbereich.

### 6. Die synergetische Wirkung des Pharmakons

Überraschender Weise wurde ein Synergieeffekt mit der erfindungsgemäßen Zusammensetzung in der Wirkung des Pharmakons festgestellt. In der Kombination der mono- oder polyvalenten spezifischen Wirkung und Zusammensetzung und immunologischen Wirkung der Eidotterantikörper (IgY) gegen pathogene Wundinfektionserreger, wie in Ziffer 2. beschrieben, mit der hervorragenden gesundheitsfördernden und Wundgewebe regenerierenden Wirkung sowie die Wundheilung deutlich beschleunigenden Wirkung der Mikroalgenextrakte gemäß Ziffer 4., ist ein sehr effektiv und schnell wirkendes Pharmakon gefunden worden. Gegenüber der separaten Anwendung von Eidotterantikörpern (IgY) und Mikroalgenextrakten von Spirulinaarten und Unterarten und/oder Chlorellaarten sowie deren möglicher Kombination mit Antibiotika werden ***Antibiotika werden Wunden, wie zahlreiche Praxisversuche ergeben haben, schneller verschlossen und gegen weitere Wundinfektionen besser geschützt.***

Nachfolgend wird die Erfindung anhand zweier Basisrezepturen näher erläutert:

### Beispiel 1

| | Gewichtsteile |
|---|---|
| Spezif. polyklonale IgY-Eidotter-Antikörper vom SPF-Huhn | 5,0 |
| Oxytetracyclinum | 2,0 |
| Ringelblumenblütenextrakt (Extr. Calendulae fluid 1:2/35%) | 4,0 |
| Mikroalgenextrakte, vorzugsweise Mikroalgenextrakte von Spirulina platensis in Form von wäßrigen (aqua), alkoholischen (ethanol.) oder CO₂-Extrakten | 2,5 |
| Paraffinum subliquidum | 25,0 |
| Lanolinum (Paraff. subliqu 15,0; Aqua 20,0; Cera lande) | ad 100,0 |

### Beispiel 2

| | Gewichtsteile |
|---|---|
| Spezif. polyklonale IgY-Eidotter-Antikörper vom SPF-Huhn | 6,0 |
| Oxytetracyclinum | - |
| Ringelblumenblütenextrakt (Extr. Calendulae fluid 1:2/35%) | 4,0 |
| Mikroalgenextrakte, vorzugsweise Mikroalgenextrakte von Spirulina platensis in Form von wäßrigen (aqua), alkoholischen (ethanol.) oder CO₂-Extrakten | 2,5 |
| Paraffinum subliquidum | 25,0 |
| Lanolinum (Paraff. subliqu. 15,0; Aqua 20,0; Cera lande) | ad 100,0 |

In den beiden Beispielen sind neben den aufgeführten Ingredienzien enthalten:
- als Antioxidans: α-Tokopherol (Vitamin E) 0,0625 %
- als Konservierungsmittel: Ascorbinsäure (Vitamin C) 0,055 %

Eine Anwendbarkeit bei Minusgraden ist wegen der leichten Verflüssigung der in den Beispielen beschriebenen Heil- und Wundsalbe gegeben.

Die Anwendungsgebiete der mit der erfindungsgemäßen Zusammensetzung hergestellten Pharmaka und Kosmetika, wie sie in den Beispielen 1 und 2 vorgegeben sind, erstrecken sich
- auf hartnäckig eiternde entzündete abszedierende gangrenös-negrodisierende Haut- und Schleimhautwunden sowie bei bakteriellen Wundinfektionen, wie beispielsweise "offene" Beine, schlecht heilende Operationsschnitte,
- auf die Verhinderung von bakteriellen Wund- Sekundärinfektionen bei Verbrennungen, Verbrühungen und Erfrierungen der Haut und der äußerlich erreichbaren mukösen Schleimhäute von Ohr, Mundhöhle, Genital- und Analbereich bei Mensch und Tier,
- zur Furunkelbehandlung sowie bei nässenden Hautekzemen von Mensch und Tier,
- zur Wundheilung bei Dekubitus,
- zur schnelleren Abheilung und antimikrobiellen Vorbeugung an Wunden aller Art sowie bei entzündlichen Haut- und kutanen Schleimhautveränderungen sowie Penisnekrosen und Kloakenentzündungen beim Wassergeflügel.

Bei der bisherigen versuchsweisen, sehr umfangreichen Anwendung der Zusammensetzung wurden Nebenwirkungen nicht bekannt.

Bei lichtgeschützter Lagerung der erfindungsgemäßen Zusammensetzung sowie einer Umgebungstemperatur von +4°C bis +8°C ist eine Haltbarkeit von mindestens 12 Monaten ab Herstellungsdatum gegeben.

Die erfindungsgemäße Zusammensetzung weist eine umfassende komplette kombinatorische Palette antiphlogistischer regenerativer antibiotischer und immunisierender Wirkungen auf.

## Patentansprüche

1. Zusammensetzung für Pharmaka und Kosmetika umfassend die Wirkbestandteile
- 4,0 bis 10,0 Gewichtsteile IgY-Eidotter-Antikörper, die gegen die relevanten Erregerstämme in infizierten Wunden oder deren Umgebung gerichtet sind, mit mono- oder polyvalenter spezifischer Wirkung aus Eidottern bzw. Dotterextrakten immunisierter SPF- und/oder Vorzugshennen unter Semi-SPF-Haltungsbedingungen,
- 2,0 bis 5,0 Gewichtsteile Mikroalgenextrakte von Spirulinaarten und Unterarten und/oder Chlorellaarten und
- 3,0 bis 5,0 Gewichtsteile Ringelblumenblütenextrakt.

2. Zusammensetzung nach Anspruch 1, **dadurch gekennzeichnet, dass** die Antikörper gegen die Erregerstämme Staphylococcus aureus, Citrobacter, Enterobacteriacege, Mikro- und Streptokokken, Corynebakterien/cornyforme (inclusive Actinomyces), coliforme (inclusive Klebsiella), Neisserien und multiresistente Staphylokokken gerichtet sind.

3. Zusammensetzung nach Anspruch 1, **dadurch gekennzeichnet, dass** sie Oxytetracyclinum enthält.

4. Zusammensetzung nach Anspruch 1, **dadurch gekennzeichnet, dass** sie Spirulinaarten und Unterarten in Form von wässrigen, alkoholischen oder CO₂-Extrakten enthält.

5. Zusammensetzung nach Anspruch 1, **dadurch gekennzeichnet, dass** sie Chlorellaarten in Form von wässrigen, alkoholischen oder CO₂-Extrakten enthält.

6. Zusammensetzung nach Anspruch 1, **dadurch gekennzeichnet, dass** sie als Mikroalgenextrakte Spirulina platensis enthält.

7. Zusammensetzung nach Anspruch 3, **dadurch gekennzeichnet, dass** sie 1,1 bis 2,5 Gewichtsteile Oxytetracyclinum enthält.

8. Zusammensetzung nach einem der Ansprüche 1 bis 7, **dadurch gekennzeichnet, dass** sie α-Tokopherol als Antioxidans enthält.

9. Zusammensetzung nach einem der Ansprüche 1 bis 8, **dadurch gekennzeichnet, dass** sie Ascorbinsäure als natürliches Konservierungsmittel enthält.

10. Verwendung von IgY-Eidotter-Antikörpern, die gegen die relevanten Erregerstämme in infizierten Wunden oder deren Umgebung gerichtet sind, mit mono- oder polyvalenter spezifischer Wirkung aus Eidottern bzw. Dotterextrakten immunisierter SPF- und/oder Vorzugshennen unter Semi-SPF-Haltungsbedingungen, Mikroalgenextrakten und Ringelblumenblütenextrakt zur Herstellung einer Zusammensetzung für Pharmaka und Kosmetika gegen hartnäckig eiternde entzündete abszedierende gangrenös-negrodisierende Haut- und Schleimhautwunden sowie gegen bakterielle Wundinfektionen, schlecht heilende Operationsschnitte, zur Verhinderung von bakteriellen Wund-Sekundärinfektionen bei Verbrennungen, Verbrühungen und Erfrierungen der Haut und der äußerlich erreichbaren mukösen Schleimhäute von Ohr, Mundhöhle, Genital- und Analbereich bei Mensch und Tier und zur Furunkelbehandlung sowie bei nässenden Hautekzemen von Mensch und Tier, zur Wundheilung bei Dekubitus, zur schnelleren Abheilung und antimikrobiellen Vorbeugung an Wunden aller Art sowie bei entzündlichern Haut- und kutanen Schleimhautveränderungen sowie Penisnekrosen und Kloakenentzündungen beim Wassergeflügel.

## Claims

1. A composition for pharmaceutical products and cosmetic products, the composition comprising:
- 4.0 to 10.0 parts by weight of IgY egg yolk antibodies which are directed against relevant germ types in infected wounds or their environment, having monovalent or polyvalent specific action from egg yolk or egg yolk extracts of immunized SPF hens and/or hens raised under semi-SPF conditions;
- 2.0 to 5.0 parts by weight of microalgae extracts selected from the group consisting of *Spirulina* species and *Spirulina* varieties and/or *Chlorella* species
- 3.0 to 5.0 parts by weight of calendula flower extract.

2. The composition according to claim 1, **characterized in that** the antibodies are directed against the germ types of Staphylococcus aureus, Citrobacter, Entereobacteriacege, Micrococcus and Streptococcus, Coryne bacteria/Coryneforme (including Actinomyces), Coliforme (including Klebsiella), Neisseria and multi-resistance Staphylococcus.

3. The composition according to claim 1, **characterized in that** further comprising oxytetracycline.

4. The composition according to claim 1, **characterized in that** the *Spirulina* species and *Spirulina* varieties are contained in the form of aqueous extracts, alcohol-based extracts, or CO₂ extracts.

5. The composition according to claim 1, **characterized in that** the *Chlorella* species are contained in the form of aqueous extracts, alcohol-based extracts, or CO₂ extracts.

6. The composition according to claim 1, **characterized in that** the microalgae extracts are *Spirulina* platensis extracts.

7. The composition according to claim 3, **characterized in that** 1.1 to 2.5 parts by weight oxytetracycline is contained.

8. The composition according to claim 1, **characterized in that** α-tocopherol as an antioxidant is contained.

9. The composition according to any one of claims 1 to 8, **characterized in that** ascorbic acid as a natural preservative is contained.

10. Use of of IgY egg yolk antibodies which are directed against relevant germ types in infected wounds or their environment, having monovalent or polyvalent specific action from egg yolk or egg yolk extracts of immunized SPF hens and/or hens raised under semi-SPF conditions; microalgae extracts and calendula flower extract for the manufacture of a composition for pharmaceutical products and cosmetic products for the treatment of resistant suppurate inflamed abscessed gangrenous-necrotic skin wounds and mucous membrane wounds as well as bacterial wound infections and surgery cuts that heal slowly, for the prevention of secondary bacterial wound infections in the case of burns, scald injuries, and frostbite of the skin and of the externally accessible mucous membranes of the ear, the oral cavity, genital and anal areas of humans and animals, for the treatment of furuncles as well as discharging skin eczema of humans and animals, wound healing of decubitus, faster healing and anti-microbial prevention on wounds of all kinds as well as inflammatory changes of the skin and cutaneous mucous membrane as well as penis necrosis and cloacal infections of waterfowl.

## Revendications

1. Composition pour agents pharmaceutiques ou agents cosmétiques comprenant les constituants actifs :
- 4,0 à 10,0 parties en poids d'anticorps de jaune d'oeuf IgY, qui sont dirigées contre les souches d'agent pathogène pertinentes dans les blessures infectées ou leur environnement, ayant un effet spécifique mono- ou polyvalent, obtenus de jaunes d'oeuf ou d'extrait de vitellus de poules immunisées sans pathogène spécifique (SPF) et/ou de qualité supérieure dans des conditions de maintien semi-SPF,
- 2,0 à 5,0 parties en poids d'extraits de microalgues des espèces de spiruline et des sous-espèces et/ou des espèces de chlorelle et
- 3,0 à 5,0 parties en poids d'extrait de fleur de souci.

2. Composition selon la revendication 1, **caractérisée en ce que** les anticorps sont dirigés contre les souches d'agent pathogène *Staphylococcus aureus, Citrobacter, Enterobacteriaceae*, micro- et streptocoques, *Corynebacterium*/coryneformes (y compris *Actinomyces*), coliformes (y compris *Klebsiella*), *Neisseria* et staphylocoques multirésistants.

3. Composition selon la revendication 1, **caractérisée en ce qu'**elle contient de l'oxytétracycline.

4. Composition selon la revendication 1, **caractérisée en ce qu'**elle contient des espèces de spiruline ou des sous-espèces sous forme d'extraits aqueux, alcooliques ou de CO₂.

5. Composition selon la revendication 1, **caractérisée en ce qu'**elle contient des espèces de chlorelle sous forme d'extraits aqueux, alcooliques ou de CO₂.

6. Composition selon la revendication 1, **caractérisée en ce qu'**elle contient en tant qu'extraits de micro-algues de la *Spirulina platensis*.

7. Composition selon la revendication 3, **caractérisée en ce qu'**elle contient 1,1 à 2,5 parties en poids d'oxytétracycline.

8. Composition selon l'une des revendications 1 à 7, **caractérisée en ce qu'**elle contient de l'α-tocophérol à titre d'antioxydant.

9. Composition selon l'une des revendications 1 à 8, **caractérisée en ce qu'**elle contient de l'acide ascorbique à titre de conservateur naturel.

10. Utilisation des anticorps de jaune d'oeuf IgY, qui sont dirigés contre les souches d'agent pathogène pertinentes dans les blessures infectées ou leur environnement, présentant une action spécifique mono- ou polyvalente, obtenus de jaunes d'oeuf ou d'extraits de vitellus de poules immunisées sans pathogène spécifique (SFP) et/ou de qualité supérieure dans des conditions de maintien semi-SFP, d'extraits de microalgues et d'extrait de fleur de souci pour préparer une composition pharmaceutique ou cosmétique agissant contre les blessures cutanées et des muqueuses, suppurantes avec ténacité, inflammatoires, nécrosantes de manière gangreneuse, formant un abcès et contre les infections bactériennes de blessure, les incisions d'opération cicatrisant difficilement, pour empêcher les infections bactériennes secondaires de la blessure en cas de brûlures, de brûlures par un liquide bouillant, de gelures de la peau et des muqueuses accessibles extérieurement de l'oreille, la cavité buccale, la région génitale et anale, chez l'homme et l'animal, et pour le traitement des furoncles ainsi qu'en cas d'eczéma suintant de la peau chez l'homme et l'animal, pour la cicatrisation en cas de décubitus, pour une cicatrisation plus rapide et une prévention antimicrobienne pour les blessures de toute sorte ainsi que dans les cas des modifications inflammatoires des muqueuses cutanées et de la peau, des nécroses du pénis et des inflammations des cloaques chez l'oiseau aquatique.
